# EUROPÄISCHE PATENTSCHRIFT

(11) **EP 1 738 172 B1**
(45) Veröffentlichungstag und Bekanntmachung des Hinweises auf die Patenterteilung: **28.11.2007**
(21) Anmeldenummer: 05716224.0
(22) Anmeldetag: 18.03.2005
(51) Int. Cl.: G01N 33/543, B81B 7/04, B01J 19/00, H01L 21/027

(54) **VERFAHREN ZUR FUNKTIONALISIERUNG VON BIOSENSOR-CHIPS**
METHOD FOR FUNCTIONALIZING BIOSENSOR CHIPS
PROCEDE DE FONCTIONNALISATION DE PUCES DE BIOCAPTEURS

(30) Priorität: 21.04.2004 DE 102004019357
(43) Veröffentlichungstag der Anmeldung: 03.01.2007
(73) Patentinhaber: SIEMENS AKTIENGESELLSCHAFT, 80333 München (DE)
(72) Erfinder: FREY, Alexander, 82024 Taufkirchen (DE); HOFMANN, Franz, 80995 München (DE); SCHINDLER-BAUER, Petra, 85591 München (DE)
(86) Internationale Anmeldenummer: PCT/EP2005/002941
(87) Internationale Veröffentlichungsnummer: WO 2005/106478

(56) Entgegenhaltungen:
- WO-A-00/75644
- WO-A-01/20330
- WO-A-01/75150
- WO-A-02/086479
- DE-A1- 10 052 165
- THEWES, R ET AL.: "Sensor arrays for fully-electronic DNA detection on CMOS" SOLID-STATE CIRCUITS CONFERENCE, 2002. DIGEST OF TECHNICAL PAPERS. ISSCC. 2002 IEEE INTERNATIONAL., Bd. 1, 3. Februar 2002 (2002-02-03), Seiten 350-473, XP002341179
- RUBINA A Y ET AL: "Hydrogel drop microchips with immobilized DNA: properties and methods for large-scale production" ANALYTICAL BIOCHEMISTRY, ACADEMIC PRESS, SAN DIEGO, CA, US, Bd. 325, Nr. 1, 1. Februar 2004 (2004-02-01), Seiten 92-106, XP004483538 ISSN: 0003-2697

## Beschreibung

Die vorliegende Erfindung betrifft ein Verfahren zur Funktionalisierung von Biosensor-Chips, insbesondere solchen auf Basis von auf einem fertig prozessierten Wafer angeordneten Halbleiter-Chips mit darauf aufgebrachten Sensorfeldern, die üblicherweise im Arrayformat angeordnet sind, und zwar eine Funktionalisierung beispielsweise mit organischen Molekülen wie z.B. Nukleinsäuren wie DNA, RNA und PNA bzw. deren Derivaten, Proteinen, Zuckermolekülen oder Antikörpern.

Biosensor-Arrays dienen zum Nachweis von Molekülen in einem zu untersuchenden Analyten. Solche Arrays werden zum Zwecke einer Miniaturisierung zunehmend auf Chips realisiert. Die Sensoren sind häufig in einer großen Anzahl auf einem Substrat angeordnet. Der hohe Grad an Parallelisierung ermöglicht eine zeitgleiche parallele Durchführung unterschiedlicher Tests, beispielsweise Tests auf das Vorhandensein unterschiedlicher Substanzen (z.B. Moleküle) in einem vorgegebenen Analyten. Aufgrund dieser Eigenschaft ergeben sich für derartige Sensor-Anordnungen, einschließlich entsprechendem Auswertesystem, vielfältige Anwendungen in der medizinischen Diagnosetechnik wie z.B. im Point-of-care oder Home-care-Bereich, in der Pharmaindustrie, (z.B. für das Pharma-Screening, Pharmakogenomics, "high troughput screening", HTS), in der chemischen Industrie, in der Lebensmittel-Analytik, in der allgemeinen Grundlagenforschung wie z.B. Gensequenzierungen, sowie der Umwelt- und Lebensmitteltechnik.

Zur Funktionalisierung solcher Biosensoren bzw. BioChips wird üblicherweise eine kleine Menge bindungsbereiter Fängermoleküle die auch als Sonden bezeichnet werden, beispielsweise eine bestimmte Nukleinsäuresequenz, auf der Oberfläche eines spezifisch gestalteten Substrats (BioChip-Grundmodul) immobilisiert. Diese Immobilisierung erfolgt üblicherweise entsprechend der eingesetzten Kopplungschemie derart an der Substratoberfläche, daß die Fängermoleküle auch bei Waschvorgängen auf dieser verbleiben. Das Grundprinzip vieler bekannter Sensoren beruht somit darauf, daß positionsspezifisch auf einem Chip zunächst solche Fängermoleküle z.B. unter Verwendung von Mikro-Dispensiertechniken aufgebracht und unter Einsatz einer entsprechenden Bindungschemie immobilisiert werden. Das Aufbringen erfolgt vorzugsweise im Arrayformat, wobei an verschiedenen Arraypositionen beispielsweise unterschiedliche Oligonukleotid-Sequenzen immobilisiert werden können. Durch möglichst kleine Abmessungen der Einzelpositionen eines Arrays kann zum einen die Arraydichte erhöht werden, zum anderen wird die Empfindlichkeit der Detektion verbessert. Mittels eines derart gebildeten DNA-Microarrays können dann beispielsweise hochgradig parallele DNA-Analysen durchgeführt werden. Die zu untersuchenden Analytmoleküle wie z.B. Nukleinsäuren werden dabei üblicherweise markiert und mit den Fängermolekülen wie z.B. Nukleinsäuren auf dem Chip hybridisiert. Eine Hybridisierung erfolgt in der Regel dabei nur zwischen exakt komplementären Nukleinsäuremolekülen. Die Intensität des gemessenen Signals ist üblicherweise zur Menge an hybridisierter Probe proportional.

Die vorgenannten Arrays werden in jüngster Zeit technisch auch in einem Biochip mit integrierter elektronischer Auswertetechnik verwirklicht. Ein solcher Biochip ermöglicht eine schnelle, einfache und kostengünstige Analyse von Biomolekülen, wie z.B. die vorgenannten Nukleinsäuren oder Proteine, in der klinischen Diagnostik und der patientenindividuellen Medizin. Ein derartiger Biochip bzw. dessen Grundmodul kann beispielsweise eine Vielzahl miniaturisierter Probenträger bzw. Sensorelemente mit in Interdigitalstruktur angeordneten Metallelektroden wie z.B. Goldelektroden, auf die jeweils Biomoleküle aufgebracht werden können, enthalten. Die Auswertung erfolgt dann über kleinste Stromverläufe. Derartige Biochips basieren auf einem Standard-CMOS-("Complementary Metal Oxide Semiconductor")-Halbleiter-Fertigungsprozeß mit zusätzlichen Goldelektroden.

Nachteilig bei der Fertigung solcher Biosensoren ist jedoch, daß bisher kein Funktionalisieren von Halbleiter-Chips, z.B. mit organischen Molekülen wie DNA oder Proteinen, auf Waferebene möglich ist, da die Funktionsschicht, d.h. die Schicht, welche die Fängermoleküle umfasst, nicht dauerhaft konservierbar ist und beim Vereinzeln bzw. Aussägen der Chips häufig zerstört wird. Zudem ist das Aufbringen einer definierten Menge an funktionstragenden Molekülen zur Funktionalisierung der Sensorfelder dahingehend problematisch, daß ein Auseinanderfließen der Flüssigkeitsmenge die Konzentration verringert und ein Ineinanderfließen ein Übersprechen des Sensorsignales zur Folge hat. Ein weiterer Nachteil liegt darin, daß vor dem Funktionalisieren eine aufwändige, aber letztlich ungenügende Reinigungsprozedur durchgeführt werden muß. Ein weiteres Problem der im Stand der Technik verfügbaren Biosensoren stellt auch das Auftreten von durch unspezifische Reaktionen hervorgerufenen Offset-Strömen beim elektrochemischen Auslesen dar.

Zur Überwindung der vorstehenden Probleme werden derzeit die Chips nach dem Vereinzeln aufwändig und von Hand einzeln gereinigt und funktionalisiert. Dieses Handling ist jedoch nicht großtechnisch realisierbar.

DE 100 52 165 A1 beschreibt ein SPR-Sensorsystem zur strukturierten Probenanordnung durch geometrische Trennung von einzelnen Sensorfeldern, die Probenmoleküle enthalten, durch Aufbringung eines Trennmittels in Form einer strukturierten Schicht auf das Sensorsystem. WO 00/075644 beschreibt eine optische Sensorplattform zum Lumineszenznachweis von Targetmolekülen durch an einzelne Sensorfelder gekoppelte biochemische Erkennungsmoleküle, wobei die Sensorfelder zur Vermeidung von optischem und/oder fluidischem Übersprechen durch Aufbringung einer Gitterstruktur und/oder einer Berandungsstruktur getrennt vorliegen. WO 01/20330 beschreibt den Aufbau und die Herstellung von Biosensoren auf der Basis von in einzelnen Sensorzellen kompartimentierten, adressierbaren Lipidbilayerschichten, wobei die Kompartimentierung durch den strukturierten Aufbau von hydrophoben Trennwänden mittels fotolithografischer Techniken oder durch Einsatz eines Polymerstempels erfolgt.

Der vorliegenden Erfindung liegt somit die Aufgabe zugrunde, insbesondere ein Verfahren bereitzustellen, das ein Funktionalisieren von Biosensoren auf Waferebene ermöglicht, ohne die vorstehend erläuterten Probleme aufzuweisen bzw. hervorzurufen. Eine weitere Aufgabe liegt in der Bereitstellung eines Biochips, der insbesondere beim Einsatz in Assay-Verfahren kein Auftreten von durch unspezifische Reaktionen hervorgerufenen Offset-Strömen beim elektrochemischen Auslesen zeigen soll.

Diese Aufgabe wird durch die in den Ansprüchen gekennzeichneten Ausführungsformen gelöst.

Insbesondere wird ein Verfahren zur Funktionalisierung eines Biosensors bereitgestellt, umfassend die Schritte:
(i) Bereitstellen eines Wafer-Substrats, das darauf angeordnet mindestens ein Sensorfeld aufweist,
(ii) Aufbringen einer Photolackschicht über die gesamte Oberfläche des Substrats und nachfolgend ein photolithographisches Strukturieren der Photolackschicht unter Verwendung einer Photomaske mit vorbestimmtem Muster, so daß eine vorbestimmt strukturierte Schicht gebildet wird, wodurch oberhalb des Sensorfelds eine entsprechende Kavität gebildet wird,
(iii) Aufbringen von Fängermolekülen auf das Sensorfeld, so daß die Fängermoleküle innerhalb der Kavität oberhalb des Sensorfelds immobilisiert werden,
wobei vor dem Schritt (iii) eine Trägerschicht auf Hydrogel-Basis, insbesondere auf Poly(meth)acrylamidgel-Basis, auf die Sensorfelder aufgebracht wird und das Aufbringen von Fängermolekülen auf die Sensorfelder in Schritt (iii) unter gleichzeitigem Aufbringen einer Trägerschicht auf Hydrogel-Basis, insbesondere auf Poly(meth)acrylamidgel-Basis, auf die Sensorfelder erfolgt, und wobei nach Schritt (iii) das Aufbringen einer Verkapselungsschicht auf Hydrogel-Basis, insbesondere auf Poly(meth)acrylamidgel-Basis, erfolgt, und weiter umfassend die Schritte des Montierens des nach Schritt (iii) derart funktionalisierten Wafers im upside-down-Modus auf eine in einem Sägerahmen gespannte Sägefolie und Aussägen der einzelnen Chips aus dem Wafer.

Im Rahmen des erfindungsgemäßen Verfahrens werden durch das Aufbringen einer vorbestimmt strukturierten Schicht, die üblicherweise auf Basis eines organischen Polymers ist, über den zu funktionalisierenden Sensorfeldern Kavitäten gebildet, die im weiteren als Kompartimente fungieren können. In diese Kompartimente können Fängermoleküle, welche zur Funktionalisierung dienen, z.B. DNA-Moleküle, eingebracht werden.

Das in Schritt (i) eingesetzte Wafer-Substrat kann ein Keramik-Substrat, ein Halbleitersubstrat (insbesondere ein Silizium-Substrat, das heißt ein Silizium-Wafer oder ein Silizium-Chip), ein Glas-Substrat oder ein Kunststoff-Substrat sein.

Gemäß einer bevorzugten Ausführungsform der vorliegenden Erfindung wird in Schritt (i) als Substrat ein fertig prozessierter Wafer mit mindestens einem Halbleiter-Chip, wobei mindestens ein Sensorfeld, üblicherweise jeweils zwei oder mehrere Sensorfelder, auf dem Halbleiter-Chip angeordnet ist bzw. sind, eingesetzt. In einer vorteilhaften Ausgestaltung des Biosensors ist die elektrische Erfassungsschaltung in dem Halbleiter-Chip integriert. Dies hat den Vorteil, daß dadurch der gesamte Meßaufbau vereinfacht werden kann, und eine höhere Meßempfindlichkeit erreicht wird. Eine weitere Vereinfachung ergibt sich durch eine Ausgestaltung, bei der die Sensorfelder auf dem Halbleiter-Chip angeordnet sind.

Als Halbleiter-Chip kann prinzipiell jedes geeignete Halbleiter-Bauelement verwenden werden. Vorzugsweise wird ein Transistorchip verwendet, der ein CMOS-Chip sein kann.

Unter "Sensorfeld" wird im Sinne der Erfindung eine Anordnung bzw. Oberfläche verstanden, auf welcher bzw. über welche die Fängermoleküle immobilisiert werden können, d.h. an welche die Fängermoleküle durch physikalische oder chemische Wechselwirkungen binden können bzw. gekoppelt sind. Diese Wechselwirkungen schließen hydrophobe, hydrophile, von der Waalsche oder ionische (elektrostatische) Wechselwirkungen und kovalente Bindungen ein. Vorzugsweise weist das Sensorfeld eine elektrisch leitende Oberfläche auf. Beispiele für geeignete Oberflächen-Materialien, die für das Sensorfeld verwendet werden können, sind Metalle wie beispielsweise Gold, Platin oder Palladium oder elektrisch leitfähige Polymere. Vorzugsweise stellt ein Sensorfeld in der vorliegenden Erfindung eine Elektrode oder einen Bestandteil einer Elektrode dar. Die Immobilisierung auf einem Sensorfeld kann in der Weise erfolgen, daß die gesamte Oberfläche eines Feldes zum Immobilisieren mit Fängermolekülen versehen wird. Es ist jedoch auch möglich, die Immobilisierung selektiv auf einzelne Bereiche/Punkte ("spots") eines Feldes zum Immobilisieren zu beschränken. Um letzteres zu erreichen, kann das Sensorfeld entsprechend ausgestaltet sein, z.B. durch chemisch für die Immobilisierung aktivierte Bereiche. In einer Ausführungsform der vorliegenden Erfindung sind die Fängermoleküle direkt, d.h. ohne die Anordnung einer weiteren Schicht wie einer nachfolgend beschriebenen Trägerschicht, an die Sensorfläche gebunden bzw. gekoppelt.

Das Sensorfeld kann ein elektrochemisches Sensorfeld oder ein Impedanz-Sensorfeld sein. Wenn das Sensorfeld ein elektrochemisches Sensorfeld ist, kann es insbesondere als Redox-Recycling-Sensorfeld eingerichtet sein. Bei einem Redox-Recycling-Sensorfeld werden zu erfassende Partikel bzw. Teilchen bzw. Targets üblicherweise mit einem Enzym-Label versehen, das nach einem erfolgten Hybridisierungsereignis mit auf dem Sensorfeld immobilisierten Fängermolekülen dazu verwendet wird, eine in die Anordnung einzubringende elektrochemisch inaktive Substanz in zwei Teilmoleküle zu spalten, von denen mindestens eines elektrochemisch aktiv ist. Dies führt zu einer Veränderung der elektrischen Eigenschaften der jeweiligen Sensor-Position im entsprechenden Biosensor-Array.

In einer besonders bevorzugten Ausführungsform der vorliegenden Erfindung sind die Sensorfelder als Interdigitalelektroden-Felder, vorzugsweise hergestellt aus Gold, Platin oder Palladium, eingerichtet.

In Schritt (ii) des erfindungsgemäßen Verfahrens wird die vorbestimmt strukturierte Schicht üblicherweise in einer Dicke im Bereich von 10 bis 300 µm aufgebracht. In Abhängigkeit von den vorgegebenen Sensorfeldern weisen die erzeugten Kavitäten bzw. Kompartiments üblicherweise einen Durchmesser im Bereich von 10 bis 300 µm auf. In der vorliegenden Erfindung erfolgt in Schritt(ii) zunächst das Aufbringen einer Photolackschicht über die gesamte Oberfläche des Substrats und nachfolgend ein photolithographisches Strukturieren der Photolackschicht unter Verwendung einer Photomaske mit vorbestimmtem Muster, wodurch oberhalb des Sensorfelds eine entsprechende Kavität gebildet wird. Die Art des Photolacks unterliegt keiner spezifischen Beschränkung. Vorzugsweise ist der Photolack jedoch ein negativer UV-Photoresist vom Epoxy-Typ. Derartige Photolacke sind unter den Handelsnamen SU-8 oder THB-430N erhältlich. Nach Belichtung mit einer entsprechend strukturierten Maske werden oberhalb der Sensorfelder entsprechende Kavitäten bzw. Kompartiments in einer Höhe bzw. Tiefe entsprechend der Dicke der Photolackschicht gebildet.

Gemäß der vorliegenden Erfindung wird vor dem eigentlichen Funktionalisieren der Sensorfelder, d.h. vor Schritt (iii), eine Trägerschicht auf Hydrogel-Basis, insbesondere auf Poly(meth)acrylamidgel-Basis, auf die Sensorfelder aufgebracht. Dabei wird das Aufbringen von Fängermolekülen auf die Sensorfelder in Schritt (iii) unter gleichzeitigem Aufbringen einer Trägerschicht auf Hydrogel-Basis, insbesondere auf Poly(meth)acrylamidgel-Basis, auf die Sensorfelder durchgeführt. Der Einsatz solcher Trägerschichten ist im Stand der Technik bekannt; siehe beispielsweise A.Yu. Rubina, Analytical Biochemistry, 325 (2004), Seiten 92-106. Dabei werden zunächst Acrylamidgruppen bzw. Methacrylamidgruppen z.B. in die terminale 3' oder 5'-Stellung von DNA-Moleküle eingeführt, gefolgt von der Polymerisation solcher Zwischenstufen, so daß ein Hydrogel gebildet wird, in welchem bzw. an welchem kovalent Fängermoleküle wie z.B. DNA-Moleküle gebunden sind. In einem solchen Fall sind die Fängermoleküle wie z.B. DNA-Moleküle nicht direkt an die Sensorfelder gebunden bzw. gekoppelt, sondern in einer entsprechenden Trägerschicht auf vorzugsweise Hydrogel-Basis über dem jeweiligen Sensorfeld immobilisiert.

In Schritt (iii) des erfindungsgemäßen Verfahrens erfolgt das Aufbringen von Fängermolekülen wie beispielsweise Nukleinsäuren, Proteinen, Zuckermolekülen, Antikörpern, etc., insbesondere Oligonukleotidsonden, auf die Sensorfelder, so daß die Fängermoleküle innerhalb der durch die vorbestimmt strukturierte Schicht, insbesondere einer entsprechend strukturierten Photolackschicht erzeugten Kavität oberhalb der Sensorfelder immobilisiert werden. Dies kann entweder auf einer geeigneten Sensoroberfläche oder mittels einer dreidimensionalen Matrix, z.B. Hydrogel, durchgeführt werden. In besonders vorteilhafter Weise kann dieser Vorgang automatisiert auf Waferebene erfolgen. Wenn das Sensorfeld in Form einer Goldelektrode vorgesehen wird, kann das Immobilisieren der Fängermoleküle unter Verwendung der aus der Biochemie bekannten, besonders vorteilhaften Gold-Schwefel-Kopplung realisiert werden, indem beispielsweise eine Thiolendgruppe der Fängermoleküle mit der Goldelektrode chemisch gekoppelt wird.

Anzumerken ist, daß es selbstverständlich möglich ist, mit dem vorliegenden Verfahren nicht nur eine einzige Art von Nukleinsäuren in einer einzelnen Meßreihe zu erfassen. Vielmehr können mehrere Nukleinsäuren gleichzeitig oder auch nacheinander erfaßt werden. Dazu können auf den Sensorfeldern mehrere Arten von Fängermolekülen, von denen jedes eine (spezifische) Bindungsaffinität für eine bestimmte zu erfassende Nukleinsäure aufweist, gebunden werden, und/oder es können mehrere Einheiten zum Immobilisieren eingesetzt werden, wobei an jeder von diesen Einheiten nur eine Art von Fängermolekül gebunden wird. Bei diesen Mehrfachbestimmungen wird für jede zu erfassende Nukleinsäure vorzugsweise eine von den anderen Markierungen unterscheidbare Markierung verwendet, um so z.B. ungewollte Nebenreaktionen zu vermeiden.
Zum Einsatz für eine solche Mehrfachbestimmung weist der Biosensor vorzugsweise mehrere, d.h. mehr als zwei, Sensorfelder zum Immobilisieren von Nukleinsäuren in einer regelmäßigen Anordnung auf.

Nach Schritt (iii) erfolgt das Aufbringen einer Verkapselungsschicht auf Hydrogel-Basis, insbesondere auf Poly(meth)acrylamidgel-Basis.

Wenn im Rahmen des erfindungsgemäßen Verfahrens ein fertig prozessierter Wafer mit darauf angeordneten Halbleiter-Chips, wobei die Sensorfelder jeweils auf den Halbleiter-Chips angeordnet sind, eingesetzt wird, kann der nach Schritt (iii) derart funktionalisierte Wafer im upside-down-Modus auf eine in einem Sägerahmen gespannte Sägefolie, wie z.B. eine für derartige Zwecke üblicherweise verwendete Mylar®-Folie, angeordnet werden, wonach das Aussägen der einzelnen Chips aus dem Wafer gemäß üblichen Techniken erfolgt. Das Sägen der Wafer erfolgt im sog. upside-down-Modus, also mit der Unterseite des Wafers nach oben. Vor der Vereinzelung der Chips kann die Folie, typischerweise nach UV-Belichtung oder Wärmebehandlung, wieder rückstandsfrei abgelöst werden, und die Funktionsschicht steht dem Anwender direkt zur Verfügung. Durch das erfindungsgemäße Aufbringen einer Photolackschicht und der daraus resultierenden Bildung von entsprechenden Kavitäten bzw. Kompartiments wird die Funktionsschicht in den Kompartimenten vor mechanischen Beschädigungen und Verunreinigungen geschützt und über einen längeren Zeitraum konserviert.

Weiterhin ist durch Verwendung des beschriebenen Verfahrens keine aufwändige chemische Reinigung der Sensorflächen (z.B. mit Cyaniden) vom Sägeschmutz mehr nötig. Ein Auseinanderfließen, resultierend in Konzentrationsverringerung, der aufzubringenden, funktionstragenden Moleküle wird ebenso durch die gemäß dem Verfahren der vorliegenden Erfindung erreichte Kompartimentierung verhindert wie ein Ineinanderfließen (Übersprechen). Durch das erfindungsgemäße Verfahren kann somit eine genau definierte Menge an funktionstragenden Molekülen in jedes Kompartiment eingebracht werden. Werden zusätzlich noch dreidimensionale Trägermaterialien wie Hydrogele verwendet, kann die Menge der funktionstragenden Moleküle pro Sensorfeld zusätzlich erhöht werden. Somit ist auch eine Verdichtung der Sensorfelder pro Fläche leichter erreichbar.

Im Rahmen der vorliegenden Erfindung wird ein Biosensor-Chip bereitgestellt, umfassend ein Substrat mit mindestens einem Halbleiter-Chip darauf angeordnet, wobei auf dem Halbleiter-Chip wiederum mindestens ein Sensorfeld angeordnet ist, das am Boden einer Kavität vorliegt, die von einer vorbestimmt strukturierten Photolackschicht umgeben ist, die vollständig über die gesamte Fläche des Halbleiter-Chips aufgebracht ist, wobei in der Kavität über dem Sensorfeld eine Funktionsschicht, umfassend Fängermoleküle, immobilisiert ist. Ein derartiger Chip kann durch das vorstehend erläuterte Verfahren hergestellt werden. Ein solcher Biosensor-Chip kann ferner einen Steuer-Schaltkreis, eine elektrische Erfassungseinrichtung bzw. einen Auswerte-Schaltkreis aufweisen. Darüberhinaus kann, wenn erforderlich, auch eine Temperaturkontroll- und regelvorrichtung wie z.B. ein Thermofühler vorgesehen werden. Diesbezüglich wird beispielsweise auf die Offenbarung von DE 102 24 567 A1, DE 102 28 125 A1 und DE 101 12 778 A1 Bezug genommen.

Die Figuren zeigen:
Fig. 1 zeigt schematisch die Herstellung einzelner Biosensor-Chips mittels einer bevorzugten Ausführungsform des erfindungsgemäßen Verfahrens.
Figuren 2, 3 und 4 zeigen schematisch das Sensorprinzip einer bevorzugten Ausführungsform eines gemäß dem Verfahren der vorliegenden Erfindung hergestellten Biosensor-Chips, wobei Fig. 2 die Draufsicht auf ein Kompartment über einer Interdigital-Goldelektroden-Anordnung zeigt, Fig. 3 schematisch zeigt, wie bei einem Biomolekülnachweisverfahren auf "Redox-Recycling"-Basis ein entsprechender Strom erzeugt wird, und Fig. 4 das bewirkte Ausgangssignal wiedergibt.
Fig. 5 zeigt schematisch das Problem der Erzeugung unspezifischer Signale, wie es sich bei Biosensoren gemäß dem Stand der Technik darstellt.
Fig. 6 zeigt schematisch, wie das Problem der Erzeugung unspezifischer Signale durch die erfindungsgemäß hergestellten Biosensoren überwunden werden kann.

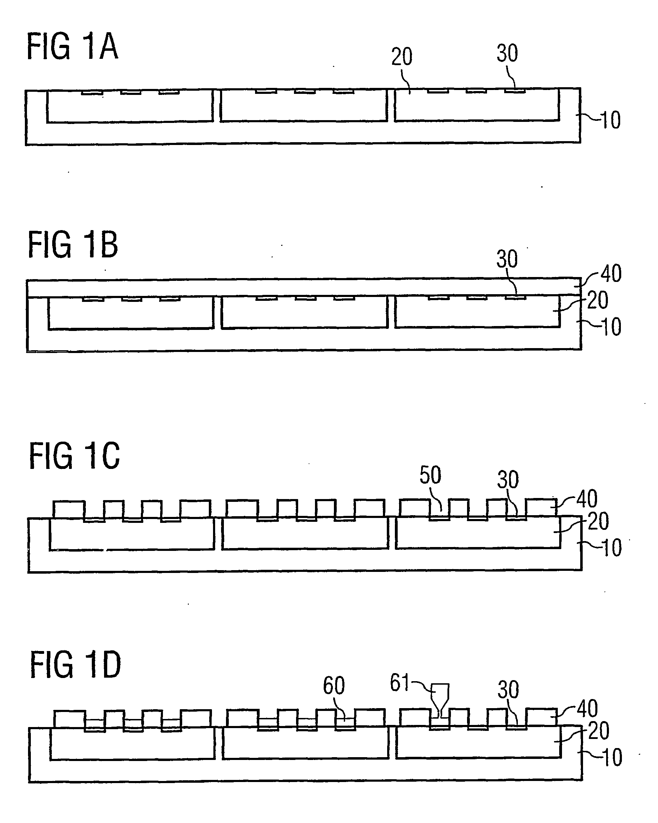

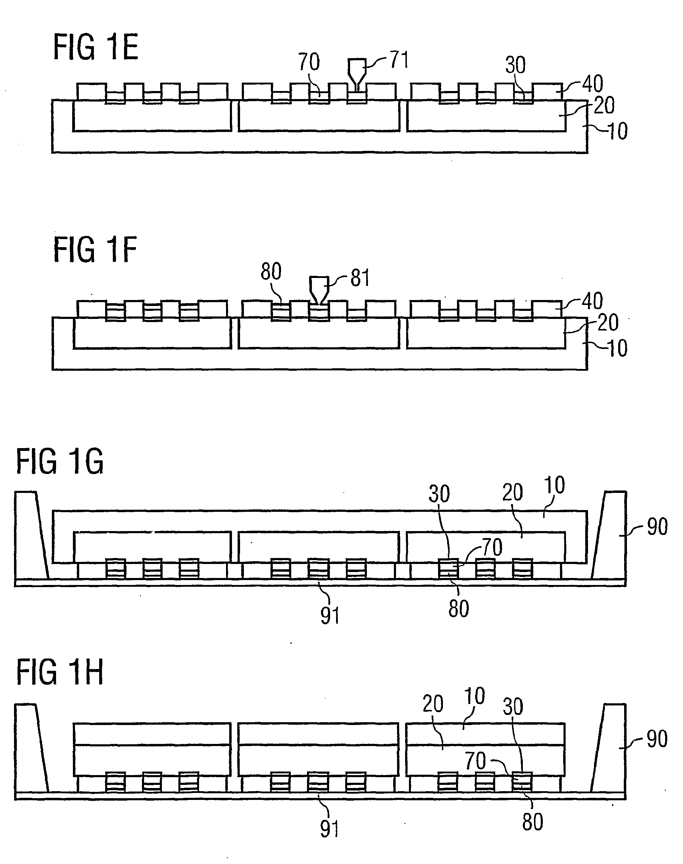

In Fig. 1 ist schematisch die Herstellung einzelner Biosensor-Chips mittels des erfindungsgemäßen Verfahrens für den Fall gezeigt, daß im Rahmen des erfindungsgemäßen Verfahrens als Substrat ein fertig prozessierter Wafer (10) mit darauf angeordneten Halbleiter-Chips (20), wobei auf den Halbleiter-Chips jeweils Sensorfelder (30) angeordnet sind, eingesetzt wird; siehe Fig. 1a). Anschließend wird über die gesamte Oberfläche des Wafers eine Photolackschicht (40) beispielsweise mittels Spincoating, etc., aufgebracht; siehe Fig. 1b). Nach der Photolackstrukturierung mittels entsprechender Photomaske (siehe Fig. 1c)), wobei Sensorfelder (60) und Sägerahmen frei belichtet werden und entsprechende Kompartiments (50) über den Sensorfelder gebildet werden, erfolgt zunächst das Aufbringen einer Trägerschicht (60) auf Hydrogel-Basis auf die Sensorfelder (30) zur Bildung einer 3D-Matrix mittels z.B. Spotting erfolgen; siehe Fig. 1d). Anschließend erfolgt die Funktionalisierung, d.h. die Immobilisierung von Fängermolekülen unter Bildung der eigentlichen Funktionsschicht (70) über den Sensorfeldern (30); siehe Fig. 1e). Daran schließt sich das Aufbringen einer Verkapselungsschicht (80) auf an Hydrogel-Basis; vgl. Fig. 1f). Das Aufbringen der Trägerschicht und der Funktionsschicht wird gleichzeitig durchgeführt. Anschließend wird der Wafer mit der funktionalisierten Seite nach unten (upside-down-Modus) auf eine in einem Sägerahmen (90) gespannte Sägefolie (91) angeordnet; siehe Fig. 1g). Abschließend werden die Biosensor-Chips aus dem Wafer ausgesägt; siehe Fig. 1h).

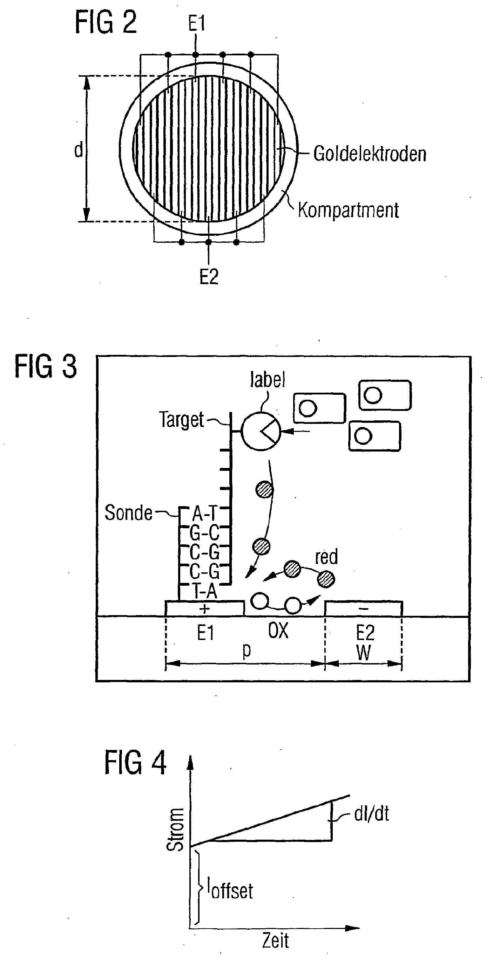

Im Folgenden werden weitere Vorteile erläutert, die sich insbesondere für elektrochemische Nachweisverfahren, bei denen Konvektions- und Diffusionsvorgänge stattfinden, ergeben. Ein Beispiel für ein solches Biomolekülnachweisverfahren ist das in Figur 3 dargestellte "Redox Recycling".

Auf jeder Oberfläche eines Pixels des Sensorfeldes ist DNA mit einer bestimmten Sequenz immobilisiert. Nach der Hybridisierung findet man Pixel mit doppelsträngiger DNA vor. Üblicherweise ist ein Label am Target-DNA Strang angekoppelt. Substratteilchen werden mit Hilfe des Enzymlabels in redoxaktive Spezies umgewandelt und diffundieren zu den Elektroden. Durch Anlegen geeigneter Potentiale wird dort ein Redox-Prozeß initiiert, der ein spezifisches Signal liefert; siehe auch R. Thewes et al, "Sensor arrays for fully electronic DNA detection on CMOS", Tech. Dig. International Solid-State Curcuits Conference (ISSCC), 2002, Seiten 350-351 und 472-473 bzw. A. Frey et al., "Design of an integrated potentiostat circuit for CMOS biosensor chips", Proc. International Symposium on Circuits and Systems (ISCAS), 2003, Seiten V9-V12.

Es ergeben sich jedoch Probleme dahingehend, daß die Redoxteilchen nicht nur spezifisch in der Nähe der sie generierenden Label zu finden sind. Hierfür kann es mehrere Ursachen geben. Ein erster Grund für das unspezifische Vorhandensein von Redoxteilchen liegt im Reaktionsgleichgewicht zwischen Substrat und Redoxteilchen.

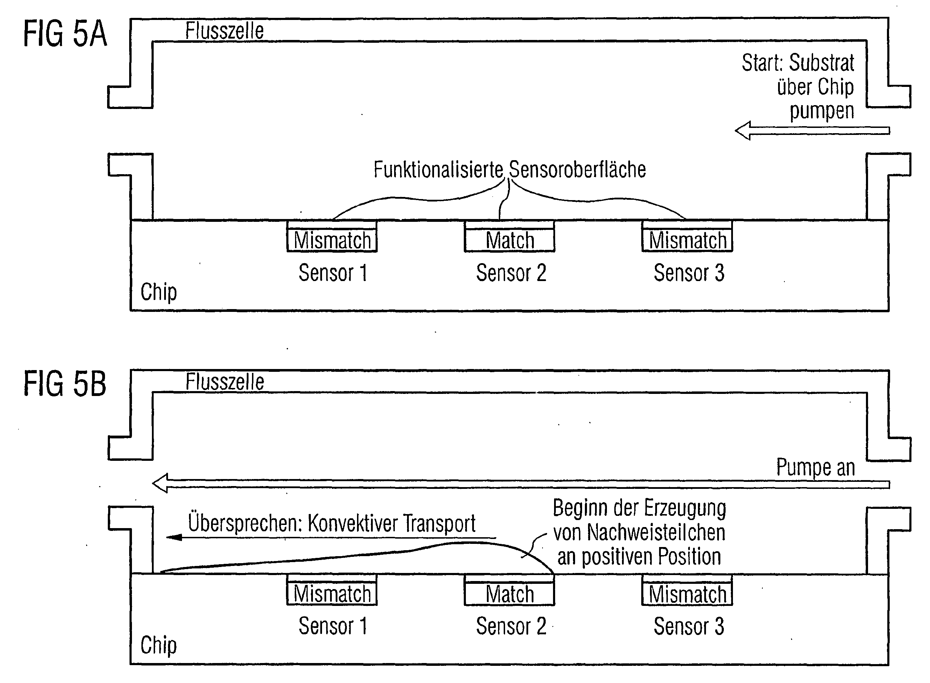

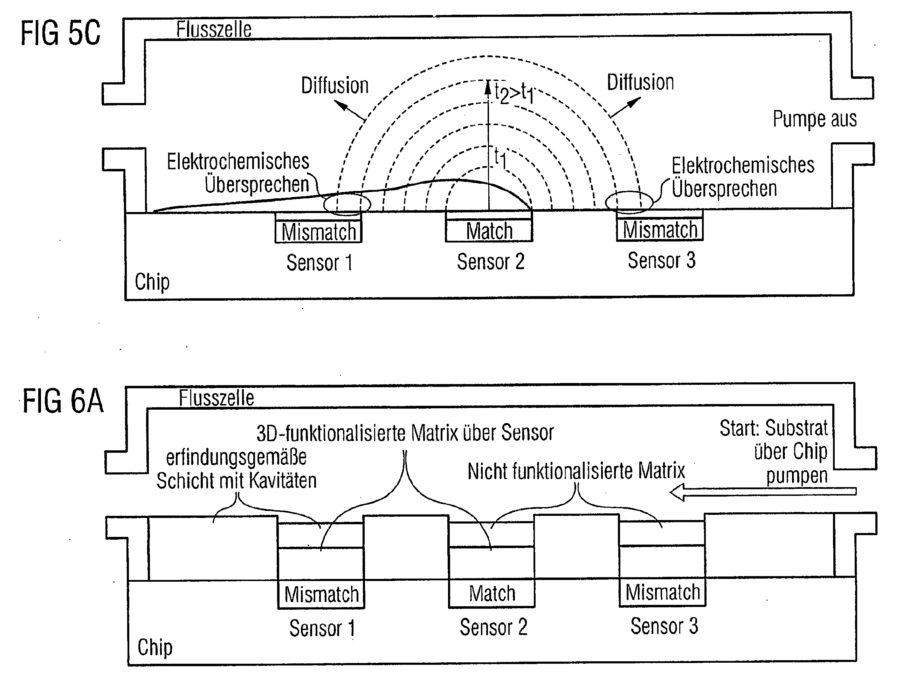

So kann unter anderem eine nicht-enzymatische spontane Hydrolyse des Substrats auftreten. Ein zweiter Grund liegt im Testablauf begründet. Die Substratteilchen werden dem Sensorchip mit einer Pumpe, also im Fluß, zugeführt (siehe Figur 5a). So bald Substrat vorhanden ist, werden an den Sensorpositionen mit einem Match (d.h. Vorhandensein von Enzymlabeln) Redoxteilchen gebildet. Diese Teilchen werden in dieser Phase durch Konvektion über den gesamten Chip verteilt und sind damit ebenfalls unspezifisch (siehe Figur 5b). Ein weiterer Grund für unspezifische Redoxteilchenkonzentrationen ist die gradientengetriebene Diffusion z.B. zwischen benachbarten Pixeln mit Match und Mismatch, wie in Figur 5c) dargestellt.

Die beschriebenen unspezifischen Redoxteilchenkonzentrationen sind besonders dann kritisch, wenn sie groß gegenüber der nachzuweisenden Konzentration sind. Es resultiert eine Gesamtkonzentration von Nachweisteilchen, die sich in einen unspezifischen Sensorstromanteil übersetzt. Dieser Offset-Anteil kann die Signalauflösung begrenzen und damit den Dynamikbereich nach unten limitieren.

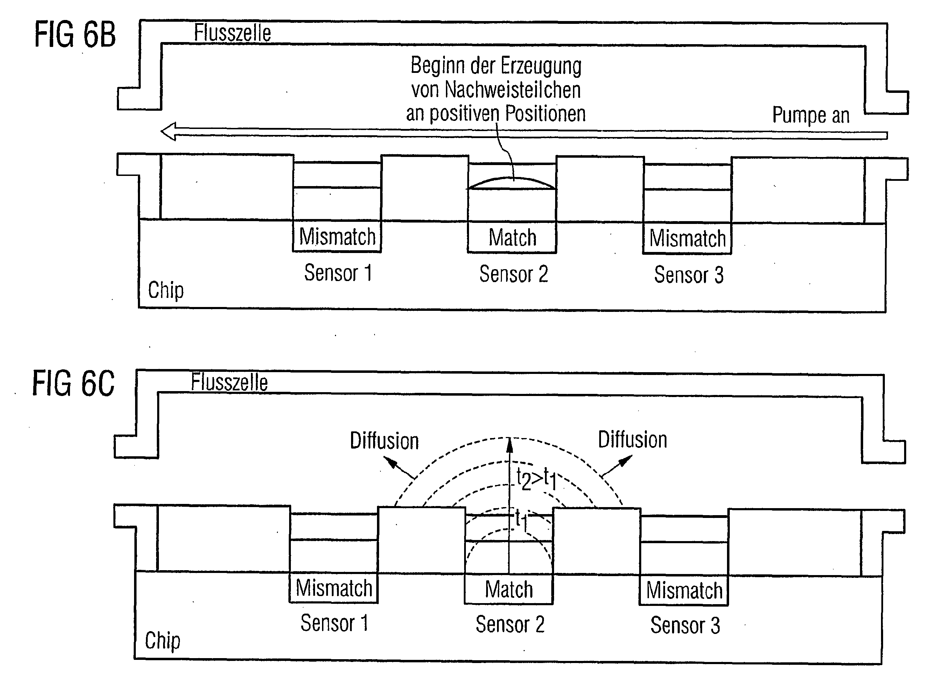

In Figur 6 ist dargestellt, wie mit Hilfe der erfindungsgemäß erzeugten Kavitäten das zuvor beschriebene Problem unspezifischer Signale vermieden bzw. deutlich reduziert werden kann. Figur 6a) zeigt eine in den Kavitäten befindliche erste Schicht (Trägerschicht), die als 3D-Funktionalisierungs-Matrix dient. Über dieser ersten Schicht befindet sich eine zweite Schicht (Verkapselungsschicht) als Diffusionsbarriere aus z.B. gleichem Material aber ohne Funktionalisierung (keine DNA-Fängermoleküle in dieser Schicht). Die zweite Schicht kann auch aus einem von der ersten Schicht verschiedenem Material bestehen, um bestimmte Eigenschaften vorteilhaft einstellen zu können (z.B. Diffusionskonstante für Substrat- und Redoxteilchen). Wird nun das Substratmaterial über den Chip gepumpt, verzögert sich das Einsetzen der Erzeugung der Redoxteilchen aufgrund der zweiten Schicht. Die Verzögerungszeit ist in gewissem Rahmen mit Hilfe der Dicke der zweiten Schicht und Materialeigenschaften, die Einfluß auf die Diffusionskonstante der Substratteilchen haben, einstellbar. Ein weiteres wichtiges Zeitintervall ist die Periode, in der sich die erzeugten Redoxteilchen noch innerhalb der Kavitäten befinden. Wird der Pumpenfluß innerhalb der Summe der zuvor beschriebenen Zeiten gestoppt, wird das unspezifische Übersprechen durch Konvektion unterbunden. Ebenfalls verhindert bzw. zumindest reduziert wird das diffusionsgetriebene elektrochemische Übersprechen, wie in Figur 6c) dargestellt. Hierfür vorteilhaft sind die diffusionsbehindernde Wirkung der zweiten Schicht und die geometrische Abschirmung der Kavitäten.

Neben der zuvor beschriebenen Unterdrückung unspezifischer Signale bewirken die Kavitäten zusammen mit den beiden Schichten auch eine Vergrößerung des Sensorsignals, da die spezifisch erzeugten Redoxteilchen räumlich besser über dem Ort der elektrochemischen Reaktion konzentriert bleiben. Ein Signalverlust durch Diffusion wird somit reduziert. Insgesamt wird also das Signal zu Rauschverhältnis verbessert.

Das erfindungsgemäße Verfahren weist zusammenfassend die folgenden Vorteile auf:
- Funktionalisierung der Sensoroberflächen automatisiert auf Waferlevel möglich
- Schutz vor mechanischen Beschädigungen
- Schutz vor Verunreinigungen
- Keine aufwändige chemische Reinigungsprozedur vor dem Funktionalisieren mehr nötig
- Konservierung der Funktionsschicht über längeren Zeitraum
- Kein Auseinanderfließen der Funktionsschicht
- Aufbringen genau definierter Molekülmengen möglich
- Kein Ineinanderfließen der Funktionsschicht und somit kein Übersprechen des Signals
- Verringerung von Dissoziation, Diffusion und Konvektion, somit Unterdrückung des unspezifischen Signalanteils
- Einbau einer künstlichen Diffusionsbarriere (Verkapselungsschicht)
- Verbesserung des Signal-Rauschverhältnisses
- Vergrößerung des Sensorsignals
- Verkleinerung der Sensorfelder möglich
- Reduktion des Offset-Signals
- Universelle Verwendbarkeit auf aktiven/passiven Chips im Arrayformat mit optischem oder elektronischem Auslesen und Beschichtung jeder Art
- adaptierbar sowohl für aktive und passive Chips als auch für Verfahren mit und ohne Label.

Das erfindungsgemäße Verfahren ist sowohl für die industrielle oder laborspezifische Herstellung von elektrisch aktiven oder passiven Halbleiter- oder anderen Chips mit Funktionsschichten, wie z.B. Nukleinsäuren, Proteinen, Zuckern, Antikörpern oder anderen chemischen und biologischen Schichten (Chemosensoren, Biochips) einsetzbar. Ebenso können die erfindungsgemäß erzeugten Kompartimente als Mini-Reaktionsgefäße, z.B. für PCR, Zellkulturzüchtung, o.ä., gegebenenfalls ausgestattet mit Temperaturkontroll- und regelvorrichtung(en) und Mikrofluidik-Rührsystem(en), eingesetzt werden.

### Bezugszeichenliste

- 10: Wafer
- 20: Halbleiter-Chip
- 30: Sensorfeld
- 40: Photolack
- 50: Kavität
- 60: Trägerschicht auf z.B.Hydrogel-Basis
- 61: Vorrichtung zum Aufbringen der Trägerschicht
- 70: Funktionsschicht, umfassend Fängermoleküle
- 71: Vorrichtung zum Aufbringen der Funktionsschicht bzw. Fängermoleküle
- 80: Verkapselungsschicht auf z.B.Hydrogel-Basis
- 81: Vorrichtung zum Aufbringen der Verkapselungsschicht
- 90: Sägerahmen
- 91: Sägefolie

## Patentansprüche

1. Verfahren zur Funktionalisierung eines Biosensors, umfassend die Schritte:
(i) Bereitstellen eines Wafer-Substrats (10), das darauf angeordnet mindestens ein Sensorfeld (30) aufweist,
(ii) Aufbringen einer Photolackschicht (40) über die gesamte Oberfläche des Substrats und nachfolgend ein photolithographisches Strukturieren der Photolackschicht unter Verwendung einer Photomaske mit vorbestimmtem Muster, so daß eine vorbestimmt strukturierte Schicht gebildet wird, wodurch oberhalb des Sensorfelds eine entsprechende Kavität (50) gebildet wird,
(iii) Aufbringen von Fängermolekülen auf das Sensorfeld, so daß die Fängermoleküle innerhalb der Kavität oberhalb des Sensorfelds immobilisiert werden,
wobei vor dem Schritt (iii) eine Trägerschicht auf Hydrogel-Basis (60), insbesondere auf Poly(meth)acrylamidgel-Basis, auf die Sensorfelder aufgebracht wird und das Aufbringen von Fängermolekülen auf die Sensorfelder in Schritt (iii) unter gleichzeitigem Aufbringen einer Trägerschicht auf Hydrogel-Basis, insbesondere auf Poly(meth)acrylamidgel-Basis, auf die Sensorfelder erfolgt, und wobei nach Schritt (iii) das Aufbringen einer Verkapselungsschicht auf Hydrogel-Basis (80), insbesondere auf Poly(meth)acrylamidgel-Basis, erfolgt, und weiter umfassend die Schritte des Montierens des nach Schritt (iii) derart funktionalisierten Wafers im upside-down-Modus auf eine in einem Sägerahmen (90) gespannte Sägefolie (91) und Aussägen der einzelnen Chips aus dem Wafer.

2. Verfahren gemäß Anspruch 1, wobei in Schritt (i) als Substrat ein fertig prozessierter Wafer mit mindestens einem Halbleiter-Chip (20), wobei mindestens ein Sensorfeld auf dem Halbleiter-Chip angeordnet ist, bereitgestellt wird.

3. Verfahren gemäß Anspruch 2, wobei in dem Halbleiter-Chip eine elektrische Erfassungsschaltung integriert ist.

4. Verfahren nach Anspruch 2 oder 3, wobei der Halbleiter-Chip ein CMOS-Chip ist.

5. Verfahren nach einem der Ansprüche 1 bis 4, wobei die Sensorfelder als Interdigitalelektroden-Felder, vorzugsweise hergestellt aus Gold, Platin oder Palladium, eingerichtet sind.

6. Verfahren nach einem der Ansprüche 1 bis 5, wobei der Photolack in einer Dicke im Bereich von 10 bis 300 µm aufgebracht wird.

7. Verfahren nach Anspruch 6, wobei der Photolack ein negativer UV-Photoresist vom Epoxy-Typ ist.

## Claims

1. Method for functionalizing a biosensor, comprising the steps of:
(i) providing a wafer substrate (10), which has at least one sensor field (30) arranged thereon,
(ii) applying a photoresist layer (40) over the entire surface of the substrate and then photolithographically patterning the photoresist layer using a photomask with a predetermined pattern, so that a layer which has been patterned in a predetermined way is formed, with the result that a corresponding cavity (50) is formed above the sensor field,
(iii) applying capture molecules to the sensor field, so that the capture molecules are immobilized within the cavity above the sensor field,
wherein prior to step (iii) a carrier layer based on hydrogel (60), in particular based on poly(meth)acrylamide gel, is applied to the sensor fields, and the application of capture molecules to the sensor fields in step (iii) is carried out with simultaneous application of a carrier layer based on hydrogel, in particular based on poly(meth)acrylamide gel, to the sensor fields, and wherein step (iii) is followed by the application of an encapsulation layer based on hydrogel (80), in particular based on poly(meth)acrylamide gel, and also comprising the steps of mounting the wafer which has been functionalized in such a manner following step (iii) in upside-down mode to a sawing sheet (91), which has been tensioned in a sawing frame (90), and sawing the individual chips out of the wafer.

2. Method according to Claim 1, wherein in step (i) the substrate provided is a fully processed wafer having at least one semiconductor chip (20), at least one sensor field being arranged on the semiconductor chip.

3. Method according to Claim 2, wherein an electrical recording circuit is integrated in the semiconductor chip.

4. Method according to Claim 2 or 3, wherein the semiconductor chip is a CMOS chip.

5. Method according to one of Claims 1 to 4, wherein the sensor fields are set up as interdigitated electrode fields, preferably made from gold, platinum or palladium.

6. Method according to one of Claims 1 to 5, wherein the photoresist is applied in a thickness in the range from 10 to 300 µm.

7. Method according to Claim 6, wherein the photoresist is a negative UV photoresist of the epoxy type.

## Revendications

1. Procédé de fonctionnalisation d'un biocapteur comprenant les stades dans lesquels :
i) on se procure un substrat (10) à tranche sur lequel est disposé au moins un champ (30) de capteurs,
ii) on dépose une couche (40) de vernis photosensible sur toute la surface du substrat et on structure ensuite photolitographiquement la couche de vernis photosensible en utilisant un masque photosensible ayant une configuration déterminée à l'avance de manière à former une couche structurée d'une manière déterminée à l'avance, une cavité (50) correspondante étant ainsi formée au-dessus du champ de capteurs,
iii) on dépose des molécules de capture sur le champ de capteurs de manière à immobiliser les molécules de capture dans la cavité au-dessus du champ de capteurs,
dans lequel avant le stade (iii), on dépose une couche support à base d'hydrogel(60), notamment à base de gel de poly(méth)acrylamide, sur les champs de capteurs et on effectue le dépôt de molécules de capture sur les champs de capteurs au stade (iii) en déposant simultanément une couche support à base d'hydrogel, notamment à base de gel de poly(méth)acrylamide, sur les champs de capteurs et dans lequel après le stade (iii) on effectue le dépôt d'une couche d'encapsulation à base d'hydrogel (80), notamment à base de gel de poly(méth)acrylamide, et comprenant, en outre, les stades du montage de la tranche ainsi fonctionnalisée après le stade (iii) en mode upside-down sur une feuille (91) de sciage tendue dans un cadre (90) de sciage et de sciage de la tranche en puces individuelles.

2. Procédé suivant la revendication 1, dans lequel au stade (i) on se procure comme substrat une tranche finie de traitement, ayant au moins une puce (20) à semiconducteurs, au moins un champ de capteurs étant disposé sur la puce à semiconducteurs.

3. Procédé suivant la revendication 2, dans lequel un circuit électrique de détection est intégré dans la puce à semiconducteurs.

4. Procédé suivant la revendication 2 ou 3, dans lequel la puce à semiconducteurs est une puce CMOS.

5. Procédé suivant l'une des revendications 1 à 4, dans lequel les champs de capteurs sont des champs d'électrodes interdigitées, de préférence en or, en platine ou en palladium.

6. Procédé suivant l'une des revendications 1 à 5, dans lequel on dépose le vernis photosensible en une épaisseur comprise entre 10 et 300 µm.

7. Procédé suivant la revendication 6, dans lequel le vernis photosensible est une réserve négative, photosensible aux UV du type époxy.
